# EUROPEAN PATENT APPLICATION

(11) **EP 2 502 989 A1**
(43) Date of publication of application: **26.09.2012**
(21) Application number: 10831184.6
(22) Date of filing: 18.11.2010
(51) Int. Cl.: C12N 5/0797, A61L 27/38, A61K 35/14

(54) **IMPLANT COMPOSITION FOR THE REGENERATION OF NEURAL TISSUE, PROCESS OF PREPARATION AND USES THEREOF**

(30) Priority: 20.11.2009 ES 200931025
(71) Applicant: Fina Biotech, S.L.U., 28023 Madrid (ES)
(72) Inventor: ZURITA CASTILLO, Mercedes, E-28223 Pozuelo de Alarcon (Madrid) (ES); VAQUERO CRESPO, Jesús, E-28223 Pozuelo de Alarcon (Madrid) (ES); AGUAYO FERRER, Concepción, E-28223 Pozuelo de Alarcon (Madrid) (ES); OTERO ORTEGA, Laura, E-28223 Pozuelo de Alarcon (Madrid) (ES)
(74) Representative: Carpintero Lopez, Francisco
(86) International application number: PCT/ES2010/070743
(87) International publication number: WO 2011/061375

(57) **Abstract**

The present invention provides a biocompatible, biodegradable graft composition for regeneration of neural tissue, comprising the following components: a) a gel scaffold formed from an isolated platelet containing fluid and an activating agent comprising calcium chloride, with or without thrombin, b) a nerve growth factor selected from BDNF, NGF, retinoic acid and combinations thereof, and c) a culture of at least 50.000 progenitor stem cells; the method of production and the uses thereof.

SEQUENCE LISTING

<110>  FINA BIOTECH S.L.U

<120>  GRAFT COMPOSITION FOR NEURAL TISSUE REGENERATION, METHOD OF
PRODUCTION AND USES THEREOF.

<130>  246/09

<160>  8

<170>  PatentIn version 3.3

<210>  1
<211>  22
<212>  DNA
<213>  Artificial

<220>
<223>  oligonucleotide

<400>  1
caggcttctc ttggctttct gg                                                22

<210>  2
<211>  19
<212>  DNA
<213>  Artificial

<220>
<223>  oligonucleotide

<400>  2
tggtgaggtt gaggtttgt                                                    19

<210>  3
<211>  21
<212>  DNA
<213>  Artificial

<220>
<223>  oligonucleotide

<400>  3
tgcgtgtgta caggtgaatg c                                                 21

<210>  4
<211>  21
<212>  DNA
<213>  Artificial

<220>
<223>  oligonucleotide

<400>  4
ggctgcatag tcatttccaa g                                                 21

<210>  5
<211>  24
<212>  DNA
<213>  Artificial

<220>
<223>  oligonucleotide

<400>  5
gtgggcaggt gggagcttga ttct                                              24


<210>  6
<211>  21
<212>  DNA
<213>  Artificial

<220>
<223>  oligonucleotide

<400>  6
ctggggcggc ctggtatgac a                                                 21


<210>  7
<211>  21
<212>  DNA
<213>  Artificial

<220>
<223>  oligonucleotide

<400>  7
cccacggcaa gttcaacggc a                                                 21


<210>  8
<211>  21
<212>  DNA
<213>  Artificial

<220>
<223>  oligonucleotide

<400>  8
tggcaggttt ctccaggcgg c                                                 21

## Description

### TECHNICAL FIELD OF THE INVENTION

The present invention is related to the field of neural tissue regeneration in the Central Nervous System (CNS). Particularly, the present invention relates to a biocompatible, biodegradable gel graft composition for regeneration of neural tissue and to methods for its manufacture and uses thereof.

### BACKGROUND OF THE INVENTION

Regeneration after injury to the Central Nervous System (CNS) is limited by inhibitory influences from the extracellular environment counteracting the repair of myelin and neurons. Often, after spinal cord or brain tissue injury, a cavity originates due to loss of native cells. This cavity is void of extracellular matrix (ECM) and usually isolated from the tissue by a glial scar, which was initially thought to block regeneration within the CNS after injury (Windle et al., J Comp Neurol. 1950; 93:241-258). More recently, researchers have demonstrated that in several different experimental models, CNS axons are capable of at least small amounts of regeneration, sprouting within the lesion site and even at short distance (Liu, CN. EI Al., Arch. Neurol. Psychiat. 1958;79:46-61*;* Guth et al., Exp Neurol. 1985;88:1-12*;* Freund et al., Nat Med. 2006;12:790-792*;* Goshgarian et al., J Appl Physiol. 2003;94:795-810*;* Li, Y. et al., J Neurosci. 1994;14:4050-4063*;* Taylor et al. J Neurosci. 2006;26:9713-9721*).* Thus, at the lesion site itself, newly formed growth cones may develop. However, they do not leave the immediate site of injury and appear incapable of producing any long-distance regeneration on their own, quickly becoming dystrophic (Li, Y. et al., Exp Neurol. 1995;134:102-111*;* Misgeld, T. et al., Nat Rev Neurosci. 2006;7:449-463*;* Misgeld, T. et al., Nat Protoc. 2007;2:263-268*).*

Strategies of neuroregeneration by cell therapy were then developed. Hematopoyetic stem cells (A.K.A mesenchimal stem cells), macrophages, dendritic cells, olfactory ensheathing cells, Schwann cells, embryonic stem cells, neural stem cells (NSCs) and neuronal progenitor cells (NPCs), have been proposed as candidates for transplantation therapy in human neural diseases (Kawasaki et al., Neuron. 2000. 28:31-40.1). However, these cells face ethical and histo-pathological challenges for their harvesting and use. In contrasts, Bone Marrow Stromal Cells (BMSCs) have the capability of differentiating into osteoblasts, adipocytes, myoblasts, chondrocytes or neurons, depending on the ECM's specific composition and factors (Engle AJ. et al., 2006. Cell 126(4): 677-689). Cells of the brain tissue can be specifically generated from BMSCs after induction with trophic factor (TF) and glial cell line-derived neurotrophic factor (GDNF). These induced BMSCs may allow for neuroregeneration. Autologous BMSCs may additionally prevent rejection-related complications (Dezawa et al., J. Clin. Invest. 2004. 113(12): 1701-1710). Indeed, isolated ex-vivo expanded autologous stem cells have been shown to integrate into host tissues following intra-CNS delivery, and differentiate in response to local cues. Despite this, transplanted cells cannot create *de novo* tissue and enter in apoptosis in response to an inappropriate interaction of cells with the ECM, known as anoikis, a type of cell death initiated by signals that are different from those that initiate other type of apoptosis, which results in a drop in cell number, below critical mass, after transplantation (Modo et al., Stroke 2002;33(9):2270-8*;* Okada, S. et al., FASEB J 2005;19:1839-41*).* In view of this, several authors have shown that the addition of trophic factors to culture medium and to biological matrices, could protect cells from apoptotic damage (Koda M. et al., Neurosci Lett 2008;444:143-7*,* Jost, M. et al., Mol Biol Cell 2001;12:1519-27*),* and may be useful for inducing cell differentiation (Silva GA., et al., Science 2004;303:1352-5). However, trophic factors did not solve the lack of ECM guidance.

Implantable scaffolds appeared as a solution to the lack of ECM support and guidance for transplanted cells. Cell-scaffold compositions, based on matrigel, collagen, alginate hydrogel, fibrin and poly-αhydroxy acid, have been tested for their usefulness in neuroregeneration of the CNS. One of the scaffolds more widely used is collagen, since it is one of the major components of the ECM and it seems to be able to induce axonal regeneration in some models of spinal cord injury (SCI) (Liu et al., Neurosurgery 2001;49:143-50*;* Goldsmith et al., Neurol Res 2005;27:115-23*).* However, collagen induces a response that leads to the sealing and isolation of the implant, rendered unable of interconnecting with the host tissue. Other scaffolds with ability to facilitate axonal elongation are alginate hydrogel (Kataoka et al., J Biomed Mater Res 2001;54:373-84*;* Suzuki et al., Neurosci Lett 2002;318:121-4*),* poly-α-hydroxy acid *(*Blacher et al., Biomaterials 2003;24:1033-40*,* Patist et al., Biomaterials 2004;25:1569-82*),* synthetic hydrogels *(*Bakshi et al., J Neurosurg Spine 2004;1:322-9*),* or polyethylene glycol (Shi R J Neurotrauma 1999;16:727-3823). Matrigel is an ECM-derived from *Engelbreth Holm Swarm* sarcoma containing laminin, fibronectin and proteoglycans. Diverse studies showed its usefulness inducing the proliferation of nerve cells *in vitro* and axonal regeneration in various models of SCI (Novikova LN. et al., J Biomed Mater Res A 2006;77:242-52*;* Xiao M., Exp Neurol 2005;194:12-30*;* Facchiano F et al., J Neurosurg 2002; 97:161-8). Fibronectin, an ECM glycoprotein, has also been successfully used as cell scaffold in CNS regeneration (Ahmed Z, Tissue Eng 2003;9:219-31). Polyglycolic Acid (PGA)-based scaffolds containing NSCs were shown to enhance reciprocal interactions between donors and transplanted cells (Park et al., Nature Biotechnology 2002; 20(11):1111-7*).* One step further in this approach, plasma polimerized alylamine (ppAAm)-treated poly(D,L-lactic acid-co-glycolic acid) particles seeded with NSCs and injected into the lesion cavity of an injured brain, gave origin to primitive '*de novo brain*' tissue which integrated efficiently with host brain tissue (Bible et al., 2009 Biomaterials 30:2985-2994). However, the degradation products of PLGA particles, and the prolonged presence of an exologous material, may exert unpredictable deleterious effects on cell physiology.

Fibrin gels (FG), prepared from fibrinogen and thrombin, are a good alternative biocompatible scaffold. Unlike the components of the extracellular matrix, FG assembles rapidly by a modified polycondensation reaction from fibrinogen (Janmey et al., 1982. Biopolymers 21, 2253-2264*).* This allows for the control of gelation times and gel morphology under physiological conditions. The biochemical and mechanical properties of fibrin have recently been exploited in numerous studies that suggest its potential for applications in medicine and bioengineering. Diverse authors have demonstrated the utility of fibrin in tissue regeneration (Falanga et al., Tissue Eng 2007;13:1299-1312*),* being occasionally used together with a growth factor (aFGF, NT-3) or in combination with other matrices (Taylor SJ et al., J Control Release 2004;98:281-9431*;* Gille J et al., Tissue Cell 2005;37:339-4832*).* FG is used in surgical procedures as a biological sealant and considered a porous scaffold that can stimulate cell adhesion and growth (Le Guéhennec L, Eur Cell Mater 2004;8:1-10).

Platelet-rich plasma (PRP) is a more sophisticated and developed implant material also containing fibrin. Their main advantage is that PRP scaffolds are manufactured from autologous materials thus, without toxicity or immune response. PRP is the autologous scaffold most often used in orthopaedic (Wakitani S, et al., J Bone Joint Surg Br 1989;71:74-80*)* and maxillofacial surgery and has shown beneficial effects on the repair of skin ulcers and as skin graft in mice (Matras et al., OsterrZ Stomatol 1970;67:338-59*).* Further, it has been used for inducing neovascularization to improve tissue perfusion in ischemic or normal tissues (US2009053208). However, at the present, there are no studies on PRP scaffolds application in the field of neurological diseases.

For a scaffold can be useful in the regeneration of a tissue, the cells that it supports should be able to acquire the phenotype of the tissue into which they are intended. In this way, many literature data show that hBMSC cultured on FG and PRP-scaffolds can be differentiated into bone or cartilage (32, 63). Furthermore, diverse *in vitro* studies support the possibility of neural BMSC-transdifferentiation (64-66), even on FG scaffolds *(*Willerth et al. "Optimization of fibrin scaffolds for differentiation of murine embryonic stem cells into neural lineage cells. Biomaterials 2006; 27:5990-6003*.),* but at present it is unknown whether this process can take place on PRP-scaffolds.

In summary, materials and strategies currently used for cell therapy in spinal cord and brain injury repair have not been completely satisfactory. A composition able of completely promoting cell multiplication, migration and differentiation into healthy neural cells is still lacking. This field needs the development of an efficient tool for CNS regeneration.

In an attempt to address this urge, the inventors of the present application, after longs experimental works, have been developed a new biodegradable, biocompatible graft composition that is able to guide and support neuroregeneration encouraging integration of the newly formed neural tissue within the existing structures.

They have shown that this new composition is advantageous when used for cerebral or spinal cord repair, since they are easier to shape, ensuring its adaptation to the injured tissue and filling posttraumatic cavities. They have demonstrated for the first time that stem cells can acquire a neural phenotype when they were seeded on these PRP-scaffolds and stimulated with growth factor, such as BDNF, NFG or retinoic acid. Thus, stem cells are capable of adhering to these biological matrices, adopting a mesenchymal morphology and showing a high survival rate.

The authors of the invention have been able to show that PRP-scaffolds can be considered as ideal matrices for the support of stem cells when these cells are used in clinical protocols applied to spinal cord injury or other traumatic lesions of the Central Nervous System.

### OBJECT OF THE INVENTION

Firstly, the invention relates to a biocompatible, biodegradable graft composition for regeneration of neural tissue, comprising a) a gel scaffold formed from an isolated platelet containing fluid and an activating agent comprising calcium chloride, with or without thrombin, b) a nerve growth factor selected from BDNF, NGF, retinoic acid and combinations thereof, and c) a culture of at least 50.000 progenitor stem cells.

Also, an object of the present invention is a method for the treatment of lesions in the central nervous system comprising grafting the composition of the invention to a subject in need thereof.

Finally, an object of the invention is a method of preparing the composition of the invention comprising i) isolation of a platelet containing fluid, ii)isolation and ex vivo expansion of progenitor stem cells, iii) combination of progenitor stem cells obtained in ii), with the platelet containing fluid obtain in i) and at least one growth factor selected from BDNF, NGF, retinoic acid and combinations thereof, iv) addition of an activating agent, comprising calcium chloride, with or without thrombin, to the combination obtained in iii), and v) gelification of the mixture obtained in iv).

### BRIEF DESCRIPTION OF THE FIGURES

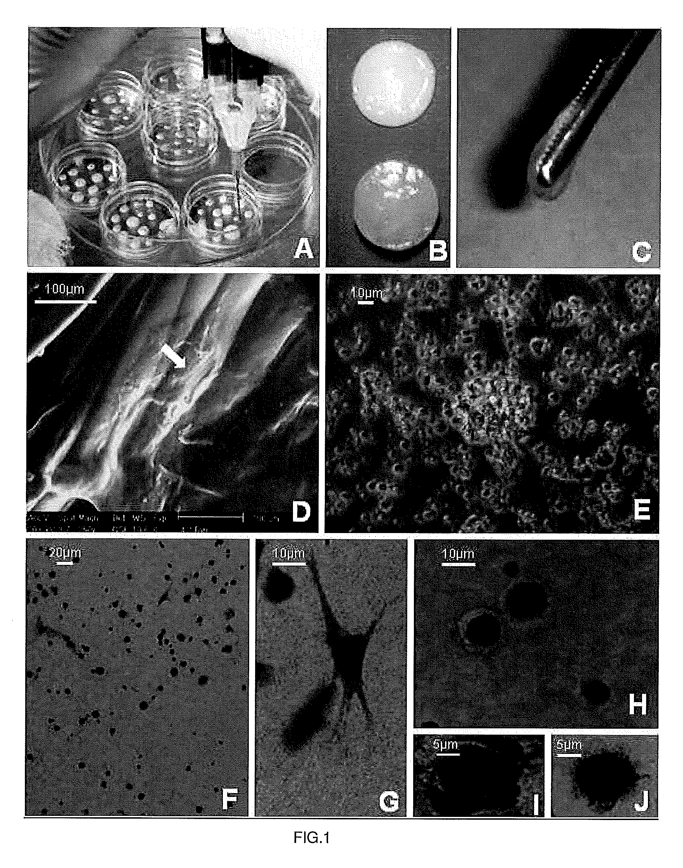

**Figure 1****. FG-scaffolds. A:** Preparation of the scaffolds used in the present study. **B:** Macroscopical aspect of the 100/50 FG-scaffold (above) and 20/100 FG-scaffold (below). Both scaffolds show a strong consistency. **C:** Macroscopical aspect of a 10/4 FG-scaffold. 10/4 and 5/2-GF scaffolds show a similar moderate consistency. **D:** Surface of a 20/100 FG-scaffold, analyzed by environmental scanning electronmicroscopy. Fibrillar structure of fibrin and some hBMSC with fibroblastic morphology (arrow), attached to the mesh of protein, can be seen. **E:** Appearance of hBMSC seeded on a 10/4 FG-scaffold. After seven days of culture, hBMSC show a spherical morphology. **F:** Appearance of hBMSC seeded on a 20/100 FG-scaffold. Cells with rounded morphology can be seen (H & E technique). **G:** Image showing an expanded hBMSC, with typical fibroblastic aspect, in a 20/100 FG-scaffold-FG (H & E technique). **H:** hBMSC seeded on a 100/500 FG-scaffold. Rounded cells, trapped in the mesh of fibrin, can be seen. (H & E technique). **I and J:** Apoptotic hBMSC on a 100/500 FG-scaffold. Typical chromatin condensation (**I**) and cytoplasmic bubbles (J) can be seen. (H E technique).

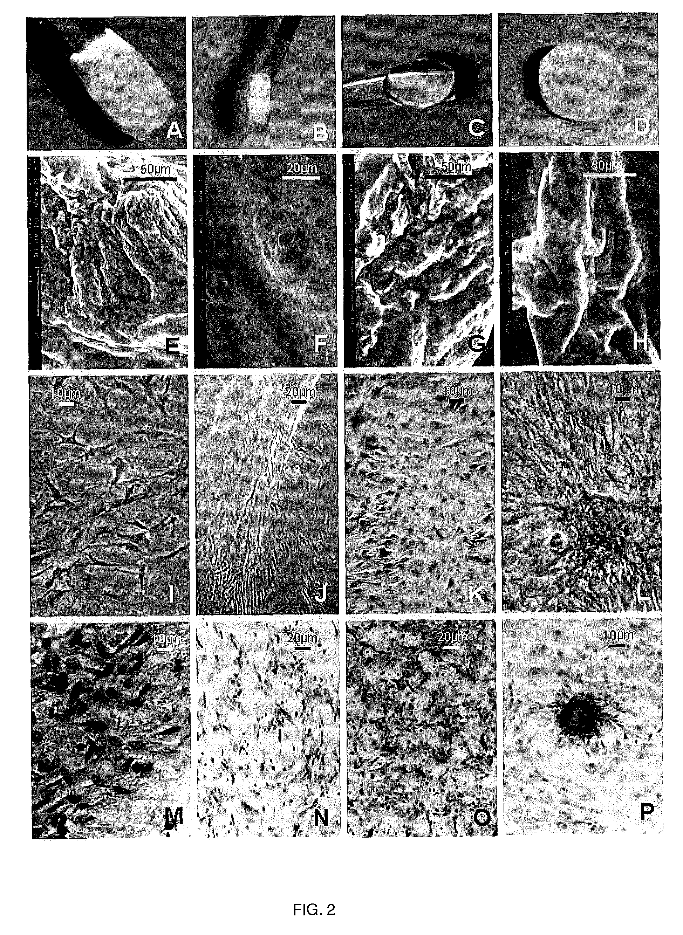

**Figure 2****.- PRP-scaffolds. A:** PRP-scaffold without thrombin. Moderate consistence. **B:** PRP-scaffold with calcium chloride and 4 IU thrombin. This PRP-scaffold and the 2 IU thrombin PRP-scaffold showed a moderate consistence. **C:** PRP-scaffold with calcium chloride and 100 IU thrombin. Gel consistency, which allows easy handling. **D:** PRP-scaffold with calcium chloride and 500 IU thrombin, showing solid but porous consistence. **E to H:** Surface of PRP-scaffolds observed by the environmental scanning electron microscope. **E:** PRP-scaffold with only calcium chloride (0 IU thrombin). Rough surface in which there is a loose and porous mesh of fibrin. It is completely covered by hBMSC and platelets, **F:** PRP-scaffold with 2 IU thrombin. The surface is smoother, but hBMSC are well bonded. **G:** PRP-scaffold with 100 IU thrombin. **H:** PRP-scaffold with 500 IU thrombin. In both types of PRP-scaffolds there is a loose mesh of fibrin, but the surface is very rough due to the number of platelets and adherent hBMSC. **I to L:** Appearance of hBMSC seeded on our PRP-scaffolds, observed by inverted microscope. The cells showed fibroblastic morphology. **I:** PRP-scaffold with only calcium chloride. **J:** PRP-scaffold with 20 IU thrombin. **K:** PRP-scaffold with 100 IU thrombin. **L:** PRP-scaffold with 500 IU thrombin. **M to P:** hBMSC cultured on our different PRP-scaffold showing fibroblastic morphology. **M:** PRP-scaffold with 20 IU thrombin. **O:** PRP-scaffold with 100 IU thrombin. **P:** PRP-scaffold with 500 IU thrombin.

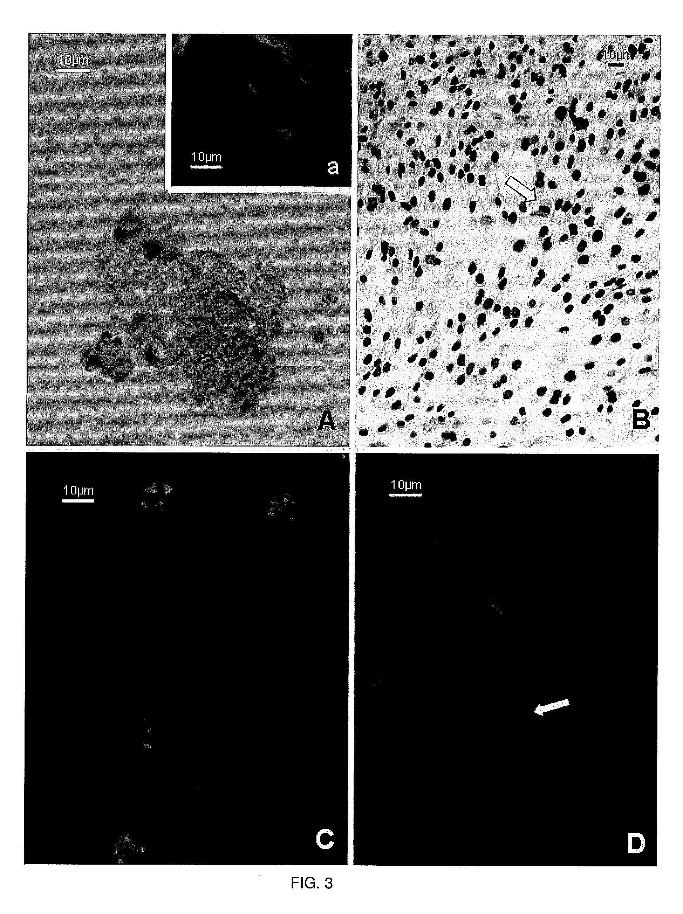

**Figure 3****.-Measuring of ability proliferation of hBMSC on FG and PRP scaffolds A:** hBMSC showing Ki-67 positivity on a 20/100 FG-scaffold. Although the cells show a spherical morphology, proliferative activity can be seen. In the insert (**a**) double-immunostain shows CD73-positive cells (green), and Ki67-expression (red). **B:** Ki-67-positive hBMSC supported on a PRP-scaffold with 100 IU thrombin. A large number of cells showing Ki-67 positivity can be seen. Arrow indicates a cell in mitosis. **Measuring of rate of cell death C:** hBMSC on a 20/100 FG-scaffold, showing TUNEL-positivity (green). D: hBMSC on a PRP-scaffold with 100 IU thrombin. Only isolated cells (arrow) show TUNEL-positivity.

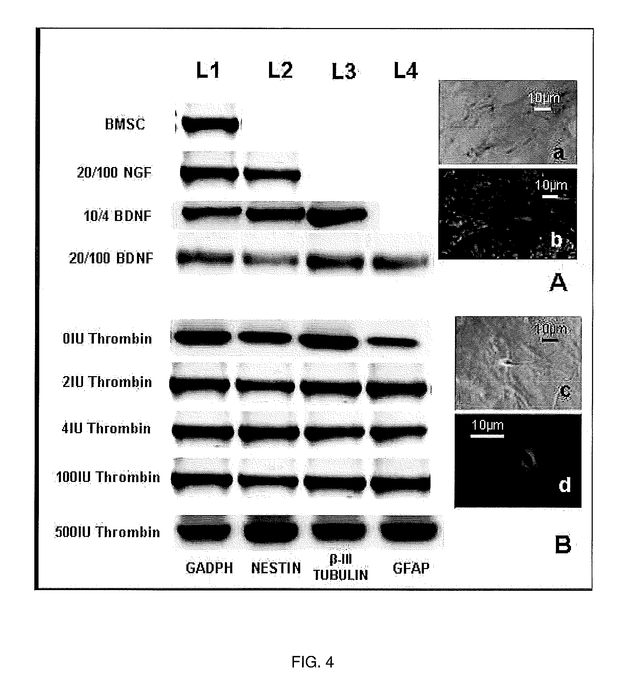

**Figure 4****.- RT-PCR analysis of cultured hBMSC. A:** Neuronal and astroglial gene expression in hBMSC seeded on FG-scaffolds. 20/100 FG-scaffold with NGF allows nestin expression, but not β-III-tubulin (neuronal) or GFAP (astroglial) gene expression. 10/4 scaffold with BDNF allows nestin and β -III-tubulin expression, but no GFAP expression. 20/100 FG-scaffold with BDNF allows expression of nestin, β - III-tubulin and GFAP. (**a**): hBMSC seeded on a 20/100 scaffold with BDNF (H.E. technique). (**b**): hBMSC seeded on a 20/100 scaffold with BDNF. Immunostain shows strong nestin-positivity. **B:** Neuronal and astroglial gene expression in hBMSC seeded on PRP-scaffolds. These scaffolds always allowed the expression of nestin, β-III-tubulin and GFAP. (**c**): hBMSC seeded on a PRP scaffold with 100 IU thrombin, showing neuronal morphology (phase-contrast microscopy). (**d**): hBMSC on a PRP.

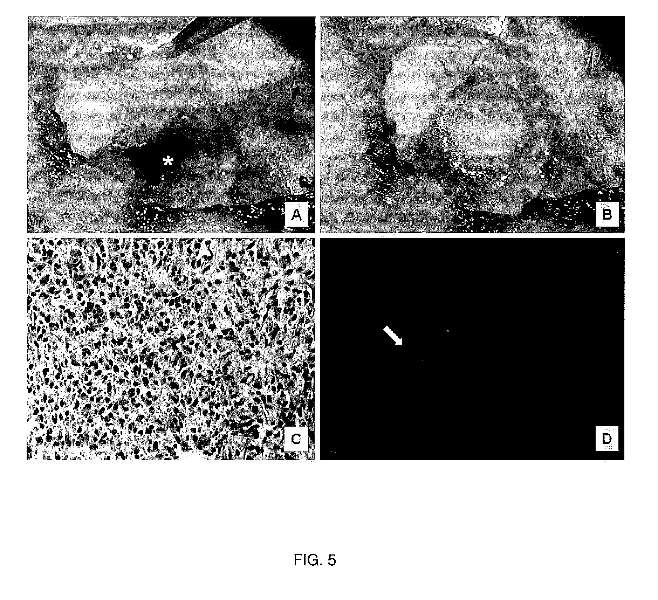

**Figure 5**.- A: Placement of the gel graft on the brain lesion zone (*). **B:** Gel graft placed on the brain lesion. **C:** aspect, one week later, of implanted cells (bisbenzamide label assays) **D:** Stromal cells labelled with bisbenzamide (cell nucleus in green) and with aspect of viable cells, seven days later, encapsulated in the gel scaffold, on the brain lesion. Inmunofluroescence shows neural differentiation of these cells (arrow) which is appreciated through the formation of cytoplasmic extensions and nestin expression (red)

### DESCRIPTION OF THE INVENTION

There is a need in the state of the art of efficient tools for the regeneration of neural tissue in lesions of the CNS.

In the present invention, the authors of the invention provide a graft composition able of completely promoting stem cell multiplication, migration and differentiation into healthy neuronal cells to be used as an efficient tool for CNS regeneration in the form of a biodegradable, biocompatible grafting scaffold that guides and supports neuroregeneration, encouraging integration of the newly formed tissue within the existing structures.

In a main embodiment, the present invention refers to a biocompatible, biodegradable gel graft composition for regeneration of neural tissue, comprising the following components: a) a gel scaffold formed from an isolated platelet containing fluid and an activating agent comprising calcium chloride, with or without thrombin, b) a nerve growth factor selected from BDNF, NGF, retinoic acid and combinations thereof, and c) a culture of at least 50.000 progenitor stem cells (hereafter, 'composition of the invention').

In the context of the present invention the following terms should be understood as follows:
Graft: an implantable element in an individual, wherein "implantable" means that it is joined with bodily tissue, typically to replace, correct or otherwise overcome a defect, comprising a scaffold and isolated cells, wherein the scaffold provides a surface suitable for adherence and proliferation of cells.
Biocompatible: refers to a compound showing compatibility with a living tissue or a living system by not being toxic, injurious, and not causing immunological rejection.
Biodegradable: refers to materials that are capable of being degraded in innocuous products and that can be gradually absorbed and/or eliminated by the body.
Bioactive: refers to a compound that causes an effect on a living organism, its parts, organs, cells or its metabolism.

The culture of isolated progenitor stem cells used in the composition of the invention can be obtained from isolated autologous or heterologous progenitor stem cells. Further, for the purpose of the present invention, the term "platelet containing fluid" is a concentration of platelets suspended in a small volume of plasma that act as a carrier of platelet growth factors, which are actively secreted by the platelets, such as platelet-derived growth factor (PDGF), platelet-derived epidermal growth factor (PDEGF), fibroblast growth factor (FGF), transforming growth factor-beta (TGF-β) and platelet derived angiogenesis growth factor (PDAF), etc.

The platelet containing fluid can be autologous or non autologous. In a preferred embodiment, the platelet containing fluid is autologous. An autologous platelet gel refers to a composition made from autologous platelet containing fluid and an autologous or non autologous activating agent. The main advantage in using autologous components in the composition is that it reduces the recipient's risk of an inflammatory response or exposure to infectious and foreign agents.

In a preferred embodiment, the platelet containing fluid is selected between platelet rich (PRP) or platelet poor plasma (PPP). In PRP, the platelet concentration is 95%, while in PPP this percentage is lower than 5%.

In a particular embodiment, when platelet poor plasma (PPP) is used, the graft composition additionally comprises platelet cell factors exogenously added. These platelet cell factors can be autologous or heterologous.

The reaction between platelets containing fluid and calcium chloride, with or without thrombin, produced the gel. Cells can be mixed ex vivo with activating agents before gelification of the scaffolds. In this way, cells are entrapped within the gel before grafting into tissue.

However, in a particular embodiment activating agents can be provided in a separate form to be applied in situ to the platelet containing fluid to form the gel scaffold in the lesion site.

In a preferred embodiment the gel scaffold is formed from a platelet rich plasma fluid and calcium chloride in an amount ranging between 25-50 µmol/l. Optionally, the composition can additionally includes thrombin in an amount ranging between 0.01-5 UI/ml.

More preferably, the growth factors used in the composition is a combination of BDNF, NGF and retinoic acid. In a more preferred embodiment, the biocompatible, biodegradable graft composition comprises BDNF in an amount ranging between 50-200 ng/ml, NGF in an amount ranging between 50-200 ng/ml and retinoic acid is in an amount ranging 0.5-2 µg/ml.

In another main embodiment, the present invention contemplates a method for the treatment of lesions in the central nervous system comprising providing the composition of the invention into the lesion site in CNS tissue in a subject in need thereof.

In a particular embodiment, the activating agent (component B) can be applied separately from the composition comprising the platelet containing fluid, the nerve growth factors and the cells (component A), into the lesion site, to form the gel scaffold '*in situ*'. In order the gel can be formed in the lesion site, the activating agents (component B) must be placed at the same time than the platelet containing fluid gel containing cells and neural growth factors (component A). In order these two components can be mixed homogenously, in the present invention a double syringe injection technique is preferably used.

In another embodiment, the present invention refers to the use of a composition of the invention in the manufacture of a pharmaceutical composition for the treatment of lesions in the CNS.

The components of the pharmaceutical composition can be administrated together or the activating agent can be administrated in a separate form from the other components

The pharmaceutical composition may include one or more pharmaceutically acceptable excipients. As used herein, a pharmaceutically acceptable excipent means a non toxic material that does not interfere with the effectiveness of the biological activity of the cells and other agents of the invention.

In another embodiment, the present invention refers to the use of a composition of the invention as a graft, for the treatment of lesions in the CNS.

In another embodiment, the present invention refers to a method of preparing the composition of the invention comprising:
i) isolation of a platelet containing fluid,
ii) isolation and ex vivo expansion of progenitor stem cells,
iii) combination of progenitor stem cells obtained in b), with the platelet containing fluid obtain in a) and at least one growth factor selected from BDNF, NGF, retinoic acid and combinations thereof,
iv) addition of an activating agent, comprising calcium chloride, with or without thrombin, to the combination obtained in c), and
v) gelification of the mixture obtained in e).

The gelification of the platelet containing fluid can be placed ex situ or in situ. In the last case, steps iv) and v) are carried out 'in situ', in the lesion site.

### EXAMPLES:

### Material and Methods

### Isolation and characterization of BMSC

Bone marrow samples were harvested from trabecular bone of hematologically normal adult patients (from 52 to 60 yr) undergoing routine total hip replacement surgery. Consent was obtained in all the cases. A single bone marrow aspirate (35-50ml) was taken from each patient's iliac crest. To prevent clotting, the aspiration needle was primed with sterile heparin sulphate (1000 U/ml). The bone marrow aspirate was aliquoted immediately after collection. Each 4 ml of the aspirate was layered onto 3 ml of steril Ficoll-Hypaque TM Plus (Stem Cell Technologies Inc., Vancouver, BC, Canada) in 15 ml conical tubes. The resulting suspension was centrifuged at 1500 rpm for 30 minutes at room temperature. The mononuclear layer at the interface between the Ficoll-Hypaque and plasma was removed and resuspended in 10ml α-MEM/2.5FBS medium and passaged through 70 µm cell strainers. The cells were centrifuged at 1000 rpm for 5 minutes and resuspended in α-MEM/2.5FBS medium. The cell number and viability were determined using the trypan-blue test, and cells were plated out at a density 5000 cells/cm² in T-75 flasks with α-MEM supplemented with 10% fetal bovine serum, 1000 units/ml penicillin, 1000 µg/ml streptomycin sulphate, 0.25 µg/ml. Cultures were carried out in a gassed incubator at 37ºC 5% CO₂. First cell culture medium replacement was carried out at day 3 to get rid of no-adherent cells. Subsequent cell culture change was performed every 2-3 days. BMSC from passage 1-2 were used thereafter. Flow cytometry analysis was used to determine specific markers on the cultured cells. Cells in suspension were mixed with fluorochrome-conjugated antibodies against CD29, CD31, CD44, CD45, CD105 and CD166 (R&D Systems SL, Inc. Minn., USA). Appropriate isotype antibodies were used to control for nonspecific staining. Staining was performed according to manufacturer's recommendations. Inmmunostained cells were acquired and analyzed on a flow cytometer (Cytomic FC 500-MPL, Beckman Coulter Inc., Fullerton, CA, USA).

### Preparation of FG-scaffolds

FG-sacaffolds were prepared using a Tissucol^{®} Duo kit 5ml (Baxter SL, Valencia, Spain) according to the manufacturer's instructions. A fibrin gel was formed by mixing two separate solutions. Solution A consisted of fibrinogen (100mg/ml), 10 IU factor XIII, 9 mg of fibronectin and 120 mg/ml of human plasminogen, dissolved in a solution of 3000 IUK bovine aprotinin, human albumin, glycine, sodium chloride, sodium citrate, Polysorbate-80, creatine and water. Solution B contained 500 IU human thrombin and 40 µmoles calcium chloride, dissolved in a solution of sodium chloride. Solution A and B were mixed in a 1:1 ratio. Solutions were prepared by dissolving fibrinogen in sterile distilled water. In our present study, four different concentration of fibrinogen were tested: 5mg/ml, 10 mg/ml, 20mg/ml, and 100 mg/ml, and four different concentration of thrombin (2 IU/ml, 4 IU/ml, 100 IU/ml and 500 IU/ml) in calcium chloride (40 µmoles/ml), were also tested.

For experiments, FG-scaffolds were prepared using various dilutions of solution A and B, and they were identified by their respective concentrations of fibrinogen and thrombin (Table I). All FG-scaffolds were polymerized using 100µl of solution A and 100 µl of solution B on P₃₅ plates, and small 200µl explants (approximately 10 explants per plate) were performed. The scaffolds was first polymerized and then incubated for 1 hour at 37 ºC, 95% relative humidity and 5% CO₂. The reaction between A and B produced a gel. We used a total of six P₃₅ plates for each type of scaffold. The hBMSC were mixed with thrombin component before scaffolds polymerization, with a total of 50,000 cells per explant. Subsequently, 3 ml of α-MEM/10%FBS complete culture medium were added to each plate. Morphological observations of cells entrapped within the gel were made with an inverted microscope daily, for seven days.

**Table I.- Final concentration of fibrinogen and thrombin solutions to prepare the FG-scaffolds**

| Fibrinogen solution (mg/ml) | Thrombin solution (IU/ml) | Scaffold (Fibrin/Thrombin) | Number P₃₅ |
|---|---|---|---|
| 5 | 2 | 5/2 | 6 |
| 10 | 4 | 10/4 | 6 |
| 20 | 100 | 20/100 | 6 |
| 100 | 500 | 100/500 | 6 |

### Preparation of PRP-scaffolds

For the preparation of PRP-scaffolds, peripheral blood from healthy donor patients was obtained and collected in tubes with sodium citrate 3.8% (0.5cc of whole blood, citrate/4.5cc). Anticoagulated blood samples were centrifuged at 2000g for 15 minutes to remove red blood cells. The supernatant was collected and a second centrifugation at 2400g for 6 minutes was performed. Each 4.5 cc the blood was collected into tubes containing 500µl sodium citrate 3.8% as an anticoagulant. The blood was centrifuged at 2500 rpm for 5 minutes and the yellow plasma containing the PRP fraction was separated. For the formation of gel, only 100µl of 10% calcium chloride (34µmoles/ml) or 50µl of 10% calcium chloride with 50µl thrombin (2, 4, 100 and 500 IU) were added per each 100 µl of PRP. All plasma-scaffolds were polymerized in P₃₅ plates made of small 200µl explants (approximately 10 explants per plate). Scaffolds was first polymerized and then incubated for 1 hour at 37 ºC, relative humidity 95% and 5% CO₂. The reaction between PRP and calcium chloride, with or without thrombin, produced a gel. Six plates P₃₅ were studied in each group. For experiments, plasma-scaffolds were prepared using various solutions (Table II). Scaffolds prepared with PRP were identified by the concentration of thrombin. The cells were mixed with the component of the thrombin or calcium chloride before polymerization of scaffolds, with a total of 50,000 cells per explant. Subsequently, 3 ml of α-MEM/10%FBS complete culture medium were added to each plate. Morphological observations of cells entrapped within the gel were made with an inverted microscope daily, for seven days.

**Table II.- Final concentrations of plasma and thrombin solutions to prepare the different PRP-scaffolds.**

| Plasma solution (µl) | Thrombin solution (IU/ml) | Cl₂Ca 34 µmoles/ml (µl) | Number P₃₅ |
|---|---|---|---|
| 100 | 0 | 50 | 6 |
| 100 | 2 | 50 | 6 |
| 100 | 4 | 50 | 6 |
| 100 | 100 | 50 | 6 |
| 100 | 500 | 50 | 6 |

### Cytological studies

Cell viability was determined by observing the cell morphology with inverted microscope and using a scale based in the study of Willerth et al. "Optimization of fibrin scaffolds for differentiation of murine embryonic stem cells into neural lineage cells. Biomaterials 2006; 27:5990-6003*.* In this scale, (-): Dead cells can be seen floating in the culture medium (all cells). (+): A few cells are alive and their morphology is rounded. (++): Many cells are alive, but only a few expansions are cytoplasmic, the rest remain round. (+++): Most of cells appear to be alive and many show a fibroblastic morphology. (++++): All cells appear to be alive and all of them showed a clear fibroblastic morphology.

To determine the degree of infiltration of cells in the matrix and the degree of cell differentiation on the basis of cytoplasmic extensions, we used the following scale (Willerth et al.): (-): Cells remain undifferentiated and most have a rounded morphology. (+): Cells remain undifferentiated and only a few showed a fibroblastic morphology. (+ +): Cells remain undifferentiated but most show a fibroblastic morphology. (+++): A few cells begin to show changes in the size of the cytoplasm, and some extend neurite-like protrusions. (++++): Most of cells show significant changes in their cytoplasm and have many extend neurite-like protrusions. (+++++): All cells show significant changes in their cytoplasm and have many extend neurite-like protrusions.

### TUNEL assay and ethidium bromide stain

For terminal deoxynucleotidyl transferase-mediated dUTP nick end-labeling (TUNEL) assay, the cultures were fixed with 4% paraformaldehyde for 15 minutes at room temperature and washed 3 times in Tris-buffered saline (TBS) buffer. The cells were then permeabilized in 1% Triton X-100 with 0.1 % sodium citrate for 15 minutes and washed 3 times in TBS buffer. Then, the presence of apoptotic cells was assayed with the TUNEL label mix (TACS XL DAB, R&D Systems, Inc. Minneapolis, USA) following the manufacturer's instructions. After a series of rinsing, nucleotides labelled with digoxigenin were enzymatically added to the DNA by terminal deoxynucleotidyl-transferase enzyme (TdT). The incubation was carried out for 60 min. The labelled DNA was detected using anti-digoxigenin peroxidase o anti-digoxigenin-FIT conjugated for 30 min. The chromogen diaminobenzidine tethrahydrochloride (DAB) resulted in a brown reaction product that was evaluated by light microscopy or fluorescence microscopy. Positive and negative controls were carried out on slides from the same block. Incubation without TdT served as the negative control. To perform the cell count at least 10 microscopical fields at 40 x were studied, the percentage of immunostained cells was recorded and averaged. We described our finding as the averaged results (mean±SD). After 7 days in culture, cell viability was measured using 50 g/ml ethidium bromide (P4170, Sigma, Madrid, Spain) to identify dead cells by their red fluorescence.

### Cell differentiation

For studies of neural differentiation, hBMSC were cultured on FG and PRP-scaffolds. FG-scaffolds with fibrinogen/thrombin 5/2, 10/4, 20/100 and 100/500 were prepared by adding the corresponding growth factor: Recombinant human Brain-Derived Neurotrophic Factor (BDNF) at 100ng/ml (Chemicon International Inc, Temecula, CA, USA), Nerve Growth Factor (NGF)-7S, at 100ng/ml (Sigma-Aldrich SL, Madrid, Spain) and Retinoic Acid (RA) at 1µg/ml (Sigma-Aldrich SL, Madrid, Spain) to the fibrinogen component. PRP-scaffolds 2 IU, 4 IU, 100 IU, and 500 IU) were prepared by adding the corresponding growth factor (NGF at 100ng/ml, BDNF at 100ng/ml, and RA at 1µg/ml) to the plasma component. All scaffolds were polymerized on P₃₅ plates performing small 200µl explants (approximately 3 explants per plate) using a total of 36 plates, three for the study of each growth factor, for each type of scaffold. The scaffolds was first polymerized and then incubated for 1 hour at 37 º C, relative humidity 95% and 5% CO₂. The cells were mixed with the thrombin component or with calcium chloride before polymerization, with a total of 50,000 cells per explant. Subsequently, 3 ml of α-MEM/10% FBS complete culture medium were added to each plate. The culture medium was renewed daily for seven days, by adding growth factors to the initial concentrations. After seven days of culture, morphological, immunocytochemical and RT-PCR studies were performed.

### Immunocytochemical analysis

To confirm hBMSC differentiation, inmunocytochemistry was performed on 7 days of culture inside of the fibrin and PRP- scaffolds. The media was removed from each scaffold and each well rinsed with 1 ml of phosphate buffered saline (PBS, pH.7.4). The cells fixed inside of the scaffold with 4% paraformaldehyde solution for 1 hour at room temperature. Then the samples were immersed in 30% sucrose buffer for 1 hour and included in OCT to be cut at 20µm under freezing. Two histological sections of each scaffold were stained using the conventional technique of hematoxylin-eosin. The cells were permeabilized by the addition of 0.1% triton-X diluted in PBS for 45 minutes. The cells were blocked using 5% goat serum diluted in PBS for 1 hour and primary antibody was applied overnight at 4ºC. Primary antibodies used were directed against mouse anti-β-III-Tubulin protein monoclonal antibody (2 µg/ml, Chemicon International Inc., Temecula, CA, USA), mouse anti-nestin monoclonal antibody (1 µg/ml, International Inc., Temecula, CA, USA), mouse anti-MAB1580 monoclonal antibody (1:200, Chemicon International Inc., Temecula, CA, USA), mouse anti-Glial Fibrillary Acid Protein (GFAP) monoclonal antibody (1 µg/ml, Chemicon International Inc., Temecula, CA, USA), and rabbit anti-Ki-67 protein monoclonal antibody (1:200, LabVision Westinghouse, Dr. Fremont, CA, USA). Subsequently, the sections were washed in PBS, and incubated with avidin-biotin-horseradish peroxidase complex (Vector Inc., CA, USA). 3',3'-Diaminobenzidine (DAB) was used as a chromogen. Control slices, lacking primary or secondary antibodies, were analyzed with each series. The sections were studied using light microscopy. Some cuts were incubated with secondary antibody Cy™ 2-conjugated anti-mouse IgG (1:200, Jackson ImmunoResearch Laboratories, Inc., Baltimore Pike, USA) and rhodamine (TRICT)-conjugated anti-rabbit IgG (1:200, Jackson) for 1 hour, at room temperature. After washing with PBS, the sections were stained with 4'6-diamidino-2-phenylindole dichlorhydrate (DAPI) (Merck KgaA, Darmstadt, Germany) and mounted with glycerol. The sections were studied using fluorescence microscopy. Control staining without primary antibody was used as a negative control. For each cell marker, at least 10 microscopical fields at 40 x were studied; the percentage of immunostained cells was averaged and recorded. We described our finding as the averaged results (mean ± SD) of 6 successive experiments using the described methodology.

### Ultrastructural studies

For transmission electron microscopy, specimens were fixed with 2.5% glutaraldeyde in a 0.06M sodium cacodylate buffer (pH 7.35) for 48-72h at 4ºC, rinsed in 0.2M sodium cacodylate buffer and postfixed with 1% osmium tetroxide at room temperature. Samples were then rinsed in 2.4% sodium chloride solution, washed in 0.2 M sodium acetate buffer (pH: 5.0) and block stained with 1% uranyl acetate in 0.2 M sodium acetate buffer (pH: 5.0) in the darkness for 30 min at room temperature. Routine procedures were followed for dehydration in alcohol and embedding in araldite. Ultrathin sections (50-70 nm) were contrasted with lead citrate and uranyl acetate, and viewed using a Philips EM-200 electron microscope. Furthermore, the surface of FG and PRP-scaffolds were studied by means of environmental scanning electronmicroscopy.

### RT-PCR studies

For RT-PCR analysis, total RNA samples were purified from cultured hBMSC using a RNA-extraction Kit (Eppendorf AG, Hamburg, Germany) according to the manufacturer's intructions and then treated to remove genomic DNA contamination using Dnase I (Bionova, Madrid, Spain). RNA concentrations were determined by measuring the absorbance at 260 nm. Complementary strand DNA was synthesized using kit RT-PCR (Eppendorf AG) according to the manufacturer's instructions. As initial denaturation was performed for 5 min at 94ºC followed by thirty cycles consisting of 45 seconds denaturation at 94ºC, 45 second primer anneling at 58ºC and 45 second of elongation at 72ºC. The run was completed with a final extension step for 5 min at 72ºC. PCR products were resolved on 1.2% agarose gel and visualized using ethidium bromide (0.5 µg/ml, Bio-Rad, Madrid, Spain). The primer sequences used for expressions of neuronal and glial specific genes were: nestin upstream 5'-CAGGCTTCTCTTGGCTTTCTGG-3' (SEQ ID NO 1), nestin downstream 5'-TGGTGAGGTTGAGGTTTGT-3' (SEQ ID NO 2), PCR product size: 431 pbs; β-III-tubulin upstream 5'-TGCGTGTGTACAGGTGAATGC-3' (SEQ ID NO 3), β-III-tubulin downstream 5'-GGCTGCATAGTCATTTCCAAG-3' (SEQ ID NO 4), PCR product size 240 pbs; GFAP upstream 5'-GTGGGCAGGTGGGAGCTTGATTCT-3' (SEQ ID NO 5), GFAP downstream 5'-CTGGGGCGGCCTGGTATGACA-3' (SEQ ID NO 6), product size 141 pbs; GADPH upstream 5'-CCCACGGCAAGTTCAACGGCA-3' (SEQ ID NO 7), GADPH downstream 5'-TGGCAGGTTTCTCCAGGCGGC-3' (SEQ ID NO 8), product size 430 pbs. The control experiments were carried out with glyceraldehyde-3-phosfate-dehydrogenase (GADPH).

### Example 1- Comparative study between fibrin gel implants and PRP gel implants in fostering cell multiplication, migration and differentiation.

### Cell morphology after seven days in culture

In the present study, hBMSC showed the following profile after phenotypic flow cytometry studies: CD29 +, CD44 +, CD105 +, CD166 +, CD34-, CD45-.

FG-scaffolds were consistent and fairly manageable, but hBMSC were not properly adhere to the fibrin mesh and a few hours of culture appeared to form clusters of cells with a rounded morphology and were only occasionally able to display any cell with a typical mesenchymal morphology (Table III).

**Table III.- Morphological aspects of the seeded hBMSC in the course of the seven days of culture, according to the scale described in the methodology (Whillert et al.).**

| **FG-scaffold:** | 5/2 scaffold | 10/4 scaffold | 20/100 scaffold | 100/500 scaffold |
|---|---|---|---|---|
| day 1 | + | + | ++ | + |
| day 2 | + | + | ++ | + |
| day 3 | + | + | ++ | + |
| day 4 | + | ++ | ++ | + |
| day 5 | ++ | ++ | ++ | + |
| day 6 | ++ | ++ | ++ | + |
| day 7 | ++ | ++ | ++ | ++ |

**Figure 1** shows different morphological aspects of FG-scaffolds and hBMSC seeded on them. 5/2 and 10/4 FG-scaffolds showed a similar moderate liquid consistency (**fig. 1****.C**). They were relatively more difficult to handle and could not be handled easily after 7 days in culture. HBMSC seeded on a 10/4 FG-scaffold show spherical morphology after 7 days of culture (**fig. 1****.E**). The cells seeded on these scaffolds were not able to join and therefore expand and proliferate, otherwise the viability did not exceed 50% ± 9.3% after staining with ethidium bromide. 20/100 FG-scaffolds had a consistency more manageable (**fig. 1****.D**), and cell viability increased to 69.5% ± 12.5%. Nevertheless, most of the cells remained with rounded morphology (**fig. 1****.F**) (H&E technique), and very few hBMSC that are expanded showed typical cytoplasmic extensions (**fig. 1****.G**) (H&E technique). 100/500 FG-scaffolds, whose consistency was very compact (**fig. 1****.B**), showed a significant increase in cell death, around 90% ± 7.8% (10% ± 2.5% viability after ethidium bromide staining), thus showing scarce or no proliferation. The cells were not stretched and in most cases they maintained a spherical morphology trapped between the networks of fibrin mesh (**fig. 1****.H**) (H&E technique). Morphologically, hBMSC had an apoptotic aspect with typically condensed nucleus and formation of apoptotic bodies (**fig. 1****.I,J**) (H&E technique). These findings were confirmed with electron microscopy, showing nuclear chromatin condensed around the nuclear membrane and a retracted cytoplasm where there were numerous small organelles and vesicles. Beside these cells were apoptotic bodies surrounded by membrane and chromatin condensed inside. Environmental scanning electron-microscopy showed a very thick fibrin mesh forming a compact structure and hindering the passage of culture medium. PRP-scaffolds containing thrombin were consistent, stable and manageable without difficulty. In all the gels observed hBMSC were properly adhered to the matrix proteins from the first day of the culture, so that a few hours of culture, cells dispersed evenly between the mesh of protein could be found (Table IV).

**Table IV.- Morphological aspect of the seeded hBMSC in the course of the seven days of culture, according to the scale described in the methodology (Willerth et al.).**

| **PRP scaffold:** | 0IU thrombin | 2IU thrombin | 4IU thrombin | 100IU thrombin | 500IU thrombin |
|---|---|---|---|---|---|
| day 1 | +++ | ++ | ++ | +++ | +++ |
| day 2 | +++ | ++ | ++ | +++ | +++ |
| day 3 | +++ | ++ | ++ | ++++ | +++ |
| day 4 | ++++ | +++ | +++ | ++++ | ++++ |
| day 5 | ++++ | ++++ | ++++ | ++++ | ++++ |
| day 6 | ++++ | ++++ | ++++ | ++++ | ++++ |
| day 7 | ++++ | ++++ | ++++ | ++++ | ++++ |

**Figure 2** shows different morphological aspects of PRP-scaffolds and hBMSC seeded on them.

These cells showed a typical mesenchymal morphology, with an expanded cytoplasm, cytoplasmic expansions and a great capacity for proliferation, a finding for which the number increased exponentially throughout the seven days of the study. At no time images consistent with apoptotic cells were observed. Viability measured by staining with ethidium bromide showed a survival of 99.5%. In ultrastructural studies, hBMSC cultured on PRP-scaffolds showed a typical morphology with rounded or ovoid nucleus, extensive endoplasmic reticulum, mitochondria and numerous cytoplasmic vesicles. In the PRP-scaffolds containig calcium chloride (34 µmoles/ml) the effectiveness of this agent as a coagulant was observed, obtaining gels with good consistency and easy to manipulate. Besides, the behavior of the cells was very similar to that observed in the scaffolds in which thrombin was used and hBMSC adhered well to the scaffold, showing an expanded cytoplasm with numerous extensions and frequent mitotic figures. The viability of cells after ethidium bromide stain was 99.5% ± 7.5%, and images consistent with apoptosis were not observed. Scaffolds formed with 2 IU and 4 IU showed a moderate consistency, too liquid to be handled satisfactorily after polymerization (**fig. 2B**). However, scaffolds polymerized with 100 IU and 500 IU were easily manipulated. Specifically, with 100 IU, gel consistency allows easy handling (**fig. 2C**) and with 500 IU, PRP-scaffold shows solid but porous consistency (**fig. 2D**). Moreover, the consistency of the gel did not appear to significantly affect cell behavior, in all cases the cell viability remained around 99% ± 6.4%, being somewhat lower in the 2 IU and 4 IU scaffolds, where the first four days of culture the cell viability fell to 68% ± 8.7%, but recovered later to reach a viability of 99% ± 12.6% at the end of the first week of culture. In these scaffolds, environmental scanning electron-microscopy showed a loose and rough surface, consisting of many protein filaments of smaller diameter than in the FG-scaffolds, and forming a relatively loose structure, allowing the entry of culture medium (**fig. 2****.E-H**). The surfaces of PRP-scaffolds were covered by hBMSC with fibroblastic morphology, and adherent protein networks and numerous platelets trapped in this network could be seen (**fig. 2****.I-P**).

### Cell viability and proliferation

The ability of proliferation of hBMSC on FG and PRP-scaffolds was measured based on the expression of Ki67 (**fig. 3****.A-B**), and the rate of cell death was assessed by TUNEL staining, after seven days of culture (**Fig. 3****.C-D**). Besides the study hBMSC by TUNEL technique, presence of cells undergoing cell death by apoptosis in the different scaffolds was always confirmed by electron microscopy. Results showed that in the case of FG-scaffolds, the percentage of TUNEL-positive cells was much higher than the percentage of cell proliferation, irrespective of the type of FG-scaffold analyzed. This difference in the balance cell death/proliferation was much more pronounced in the case of 5/2 and 100/500 FG-scaffolds (Table V).

**Table V.- Percentage of hBMSC showing TUNEL and Ki-67-positivity on the different FG-scaffolds**

| **FG-scaffold:** | 5/2 scaffold | 10/4 scaffold | 20/100 scaffold | 100/500 scaffold |
|---|---|---|---|---|
| TUNEL (%) | 71±6.4 | 68±3.2 | 63±9.5 | 80±7.8 |
| Ki-67 (%) | 1.9%±0.2 | 2.3%±0.9 | 5%±4.1 | 0.8±0.2 |

On the other hand, the balance proliferation/apoptosis for hBMSC seeded on PRP-scaffolds inclined towards side of the proliferation, being much smaller the number of TUNEL-positive cells. The hBMSC cultured on PRP-scaffolds showed a high rate of proliferation (87.9% ± 16.7% to 99% ± 13.7%) being slightly higher in the cases of 0 IU, 100 IU and 500 IU scaffolds (around 99%) compared with the 2 IU and 4 IU PRP-scaffolds (around 87%). Similarly, the number of TUNEL positive cells was very small (2.1 % ± 4.9 %) (Table VI).

**Table VI.- Percentage of hBMSC showing TUNEL and Ki-67-positivity on the different PRP-scaffolds**

| **PRP-scaffold:** | 0IU thrombin | 2IU thrombin | 4IU thrombin | 100IU thrombin | 500IU thrombin |
|---|---|---|---|---|---|
| TUNEL (%) | 0.89±0.13 | 2.1%±4.9 | 1.7%±0.3 | 0.3%±0.01 | 0.8%±0.02 |
| Ki-67 (%) | 95%±13.2 | 89%±15.1 | 87.9%±16.7 | 99%±8.95 | 99%±13.7 |

### Cell differentiation

The differentiation state of hBMSC in FG-scaffolds over 7 days was assessed using morphology and immunocytochemistry. To confirm that the hBMSC were differentiating into neural phenotypes, these cells were stained for nestin, β-III-tubulin and GFAP.

After seven days of culture in presence of different growth factors, hBMSC-seeded on FG-scaffolds showed scarce signs of differentiation, due to poor survival of these cells (Table VII).

**Table VII.- Morphological changes in the hBMSC seeded on the FG-scaffolds, after induction of differentiation by the different growth factors. Seven days of culture. Morphology was recorded according to the scale described in the methodology (Willerth et al.).**

| **FG-scaffold:** | 5/2 scaffold | 10/4 scaffold | 20/100 scaffold | 100/500 scaffold |
|---|---|---|---|---|
| NGF | - | ++ | +++ | - |
| BDNF | - | +++ | ++++ | - |
| RA | - | + | + | - |

When FG-scaffolds were treated with NGF, morphological changes consistent with neural differentiation of the seeded h-BMSC were not generally observed. In the 10/4 GF-scaffolds hBMSC were not as rounded as in conventional cultures, despite the absence of morphological changes of neural differentiation, and they appeared to adhere more to the scaffold, expanding and acquiring a fibroblastic morphology. In the 20/100 FG-scaffolds treated with NGF, some cells (10% living cells) showed slight changes in their cytoplasm and some cytoplasmic expansions that were interpreted as neurite-like expansions. The rest of the cells remained with a typical fibroblastic morphology. In the 5/2 and 100/500 FG-scaffolds, there was no sign of neural differentiation at this time, and the hBMSC maintained a rounded morphology. Nevertheless, the treatment with BDNF showed better results. In FG 10/4 FG-scaffolds, a small percentage of cells (11.2% ± 3.4% of living cells) began to show changes in the size of their cytoplasm and to present some neurite-like protrusions. In the 20/100 FG-scaffolds most cells (70.6% ± 15.6% of living cells) showed signs of neural differentiation such as cytoplasmic shrinkage and a significant presence of numerous neurite-like protrusions. In the 5/2 and 100/500 FG-scaffolds, there was no sign of neural differentiation, and the hBMSC maintained a rounded morphology.

When FG-scaffolds were treated with RA, hBMSC showed few signs of neural differentiation, after the same time of culture. In the 10/4 and 20/100 FG-scaffolds a small percentage of cells (5.6% ± 2.4% of living cells) showed a fibroblastic morphology, but the rest remained undifferentiated with rounded morphology. In the 5/2 and 100/500 FG-scaffolds hBMSC remained as undifferentiated cells, most of them showing a rounded morphology. Immunohistochemical studies confirmed the morphological findings of neural differentiation. Table VIII shows the percentage of cells expressing nestin, β-III-tubulin and GFAP.

**Table VIII.- Neural differentiation of hBMSC on our different FG-scaffolds, in presence of NGF, BDNF and RA. Seven days of culture.**

| **FG- Scaffold:** | **5 /2 scaffold** | **10/4 scaffold** | **20/100 scaffold** | **100/5scaffold** |
|---|---|---|---|---|
| **FG + NGF** | | | | |
| Nestin | - | - | 93.8 ± 2.3 | - |
| β-III-tubulin | - | - | 6.2 ± 1.7 | - |
| GFAP | - | - | 0 | - |

| **FG + BDNF** | | | | |
|---|---|---|---|---|
| Nestin | - | 13.2 ± 5.6 | 75 ± 5.2 | - |
| β-III-tubulin | - | - | 20 ± 9.4 | - |
| GFAP | - | - | 5 ± 3.4 | - |

| **FG + RA** | | | | |
|---|---|---|---|---|
| Nestin | - | - | - | - |
| β-III-tubulin | - | - | - | - |
| GFAP | - | - | - | - |

Immunocytochemical expression of MAB 1580 was absent, suggesting absence of oligodendroglial differentiation. After seven days of culture, the PRP-scaffolds treated with NGF showed an overall high percentage of cells with signs of neural differentiation, with some shrinkage of the cytoplasm and neurite-like protrusions. These results were more relevant in the PRP-scaffolds with 0 IU, 100 IU and 500 IU of thrombin, where 78.2% ± 6.9% of cells showed significant signs of neural differentiation. In the PRP-scaffolds with 2 IU and 4 IU thrombin, most cells remained undifferentiated and after seven days of culture, only a very small percentage of them (4.91% ± 1.5%) began some signs of neural differentiation in the PRP-scaffolds with 4 IU thrombin. At this time, all hBMSC showed morphological signs of neural differentiation in the PRP-scaffolds with 100 and 500 IU of thrombin and treated with BDNF, while 70.6% ± 4.9% of hBMSC showed neural differentiation in the PRP-scaffolds with 0 IU and 4 IU thrombin, and 6.21% ± 4.6% of hBMSC showed neural differentiation in the PRP-scaffolds with 2 IU thrombin. The results in the group of PRP-scaffolds treated with retinoic acid were different. In the scaffolds with 100 and 500 IU of thrombin, a rate of 68.9% ± 7.85% of cells with significant morphological changes of neural differentiation, were found. In the PRP-scaffolds with 0 IU and 4 IU thrombin, changes consistent with neural differentiation were observed in 5.3% ± 2.3% of cells. In the PRP-scaffolds with 2 IU thrombin, few signs of neural differentiation were found, remaining most of the cultured hBMSC with a fibroblastic morphology (Table IX).

**Table IX.- Morphological changes in the hBMSC seeded on our PRP-scaffolds, after induction of differentiation by the different growth. Seven days of culture. Morphology was recorded according to the scale described in the methodology (Willerth et al).**

| **PRP-scaffold:** | 0IU thrombin | 2IU thrombin | 4IU thrombin | 100IU thrombin | 500IU thrombin |
|---|---|---|---|---|---|
| NGF | ++++ | ++ | +++ | ++++ | ++++ |
| BDNF | ++++ | +++ | ++++ | +++++ | +++++ |
| RA | +++ | ++ | +++ | ++++ | ++++ |

The morphological changes consistent with the acquisition of a neural phenotype were accompanied by the expression of immunohistochemical markers, such as nestin, β-III-tubulin and GFAP. The inmunocytochemical expression of MAB 1580 was absent, suggesting absence of oligodendroglial differentiation. Table X shows the percentage of immunostained hBMSC depending on the growth-factor used.

**Table X.- Neural differentiation of hBMSC on our different PRP-scaffolds, in presence of NGF, BDNF and RA. Seven days of culture.**

| **PRP-scaffold:** | **0 IU thrombin** | **2 IU thrombin** | **4 IU thrombin** | **100 IU thrombin** | **500 IU thrombin** |
|---|---|---|---|---|---|
| **PRP + NGF** | | | | | |
| Nestin | 10.2±5 | 2.7±0.5 | 2.7±0.14 | 9±6.4 | 7.2±1.8 |
| β-III-tubulin | 17.1±6.2 | 8.61±3.1 | 12.2±5.7 | 25.6±5.9 | 39.6±5.6 |
| GFAP | 14.2±3.2 | 3.1±0.65 | 6.81±3.6 | 18.9±6.7 | 24.7±6.5 |

| **PRP+BDNF** | | | | | |
|---|---|---|---|---|---|
| Nestin | 9.45±3 | 4±2.1 | 6.1±2.4 | 1±2.7 | 12.1±2.8 |
| β-III-tubulin | 19.2±3.5 | 9±5.3 | 12.6±4 | 27.2±5.9 | 40.8±3.27 |
| GFAP | 14.7±2.1 | 6±2.5 | 4.3±0.1 | 20.2±6 | 29.6±8.6 |

| **PRP+RA** | | | | | |
|---|---|---|---|---|---|
| Nestin | 5.1±0.1 | 3±2.4 | 6.4±2.1 | 9.1±6.2 | 8.5±0.4 |
| β-III-tubulin | 12.3±7.5 | 9.2±3.5 | 10.9±3.7 | 31.2±7.8 | 29±8.6 |
| GFAP | 4.2±3.4 | 1.9±0.8 | 3.41±2.6 | 8.6±3.5 | 11.2±2.9 |

To confirm these results, RT-PCR analysis of cultured hBMSC were performed, and the results showed strong correlation with morphological and immunocytochemical findings. One week after treatment, presence of nestin-mRNA, and strong neuronal (β-III-tubulin) and astroglial (GFAP) mRNA expression, were found (Figure 4).

### Example 2

A total of 10 adult Wistar rats were used. All of them were submitted to a cortical lesion, through brain cortex resection at the parietal level after craniotomy. Immediately after the lesion, blood was extracted from the animals and gel was prepared according to the protocol:
Component A: Platelet Rich plasma (PRP) + cells (500.000) + BDNF (100 ng/ml)
Component B: Thrombine 5 UI + calcium chloride (34 micromoles/ml).

Both components were mixed and the resulting gel was placed on the brain lesion in 6 animals (fig. 5A and B). In 4 animals, the same protocol was carried out, but without cells.

After a week, histological studies were carried out. In figure 5C the aspect of grafted cells are shown (study developed through bisbenzimide label analysis). It was observed that grafted cells showed a normal aspect and their viability was higher than 60%. In those animals in which cells encapsulated in the gel were implanted, viability of grafted cells with neural differentiation signals were observed (nestin positivity). In figure 5D the immunofluorescence shows neural differentiation of these cells (arrow) which is appreciated through the formation of cytoplasmic extensions and nestin expression (red). Bisbenzamide is a nuclear label which allows identifying labelle stem cells in order to see their viability. Nestin is a cytoplasmic label which show a early neural differentiation.

In those animals wherein gel without cells was grafting, the histological result showed the typical image of a brain contusion zone, with macrophages and gliosis.

## Claims

1. A biocompatible, biodegradable graft composition for regeneration of neural tissue, comprising the following components:
a. a gel scaffold formed from an isolated platelet containing fluid and an activating agent comprising calcium chloride, with or without thrombin,
b. a nerve growth factor selected from BDNF, NGF, retinoic acid and combinations thereof, and
c. a culture of at least 50.000 progenitor stem cells.

2. A biocompatible, biodegradable graft composition according to claim 1, wherein the activating agent can be provided in a separate form from platelet containing fluid to form in situ the gel scaffold.

3. A biocompatible, biodegradable graft composition according to claim 1, wherein the platelet containing fluid is selected between platelet rich plasma and platelet poor plasma.

4. A biocompatible, biodegradable graft composition, according to claim 3, wherein the platelet containing fluid is platelet poor plasma.

5. A biocompatible, biodegradable graft composition, according to claim 4, further including platelet cell factors exogenously added.

6. biocompatible, biodegradable graft composition according to claim 3 wherein a) is a gel scaffold formed from a platelet rich plasma fluid and calcium chloride in an amount ranging between 25-50 µmol/l.

7. A biocompatible, biodegradable graft composition according to claim 6, wherein a) additionally includes thrombin in an amount ranging between 0.01-5 Ul/ml.

8. A biocompatible, biodegradable graft composition according to any of claims 6 and 7 wherein b) is a combination of BDNF, NGF and retinoic acid.

9. A biocompatible, biodegradable graft composition, according to claim 8, wherein BDNF is in an amount ranging between 50-200 ng/ml, NGF is in an amount ranging between 50-200 ng/ml and retinoic acid is in an amount ranging 0.5-2 µg/ml.

10. Use of a composition according to any of claim 1-9 as a graft.

11. Use of a composition according to claims 1-9, in the manufacture of a pharmaceutical composition for the treatment of lesions in the CNS.

12. A method of preparing a composition, according to claim 1, comprising:
i. isolation of a platelet containing fluid,
ii. isolation and ex vivo expansion of progenitor stem cells,
iii. combination of progenitor stem cells obtained in ii), with the platelet containing fluid obtain in i) and at least one growth factor selected from BDNF, NGF, retinoic acid and combinations thereof,
iv. addition of an activating agent, comprising calcium chloride, with or without thrombin, to the combination obtained in iii), and
v. gelification of the mixture obtained in iv).
